# EUROPEAN PATENT APPLICATION

(11) **EP 4 785 882 A1**
(43) Date of publication of application: **05.08.2026**
(21) Application number: 25155165.1
(22) Date of filing: 31.01.2025
(51) Int. Cl.: A61B 17/32, A61B 17/3205

(54) **CATHETER-PUNCH ASSEMBLY**

(71) Applicant: Maslanka Patentverwaltung GmbH, 78532 Tuttlingen (DE)
(72) Inventor: MASLANKA, Herbert, 78532 Tuttlingen (DE)
(74) Representative: Weickmann & Weickmann PartmbB

(57) **Abstract**

The present invention relates to a catheter-punch assembly (10), comprising a hollow outer tube portion (12) extending in a longitudinal direction (L) between a first end and a second end (16), a cable element (18) extending inside the tube portion (12) in a slidable manner, an actuation element provided at the first end of the tube portion (12) and adapted to cause a sliding motion of the cable element (18) between a first position and a second position upon actuation by a user; and a punch element (100), which is connected to or formed integrally with the cable element (18), comprising a base portion (102) adjacent to the cable element (18), a bridge portion (104) extending from the base portion (102) at a section of its circumference in the longitudinal direction (L), and a head portion (108) comprising a first longitudinal section (110) extending from the bridge portion (108) in the longitudinal direction (L), a second longitudinal section (112) opposite to the first longitudinal section (110) and extending along a part of the longitudinal extension of the first longitudinal section (110), and a longitudinal end section (114) connecting the first and second longitudinal sections (110, 112), wherein a single punch section (116) is provided between the first and second longitudinal sections (110, 112), with a clearance (118) further provided between the first and second longitudinal sections (110, 112), wherein in the first position of the cable element (18), the punch section (116) of the punch element (100) is located outside the second end (16) of the tube portion (12), and in the second position of the cable element (18), the punch section (116) is located inside the tube portion (12).

## Description

The present invention relates to a catheter-punch assembly comprising a hollow outer tube portion, a cable element extending inside the tube portion, an actuation element and a punch element, as well as to a system comprising such an assembly and a tubular or wire-shaped element to be punched by the punch element.

Examples of such assemblies are generally known in the art and are used to punch or cut tubular or wire-shaped elements inside the bodies of patients, for example inside the abdomen. In corresponding assemblies known in the art, actuatable punch elements are used, which comprise a head portion from which two opposing punch sections are extending in an oblique or slanted manner in order to facilitate the punching operation.

Thus, when a tubular or wire-shaped element is punched by such an assembly, a piece of the punched element located between the two opposing punch sections remains as a cutoff or scrap and may be pulled into the tube element at its corresponding distal end. Thus, in particular in cases in which tubular elements are punched with such an assembly known in the art, there is a risk that a corresponding scrap will be caught between the punch element and the outer tube element, and the assembly might get jammed.

Even though in alternative use cases in which wire-shaped elements or sutures are being punched or cut with a corresponding punch element, the resulting cutoffs will probably not jam the device, they nevertheless might get lost in the corresponding patient, which should be avoided in surgical procedures.

It is therefore an object of the present invention to improve upon known catheter-punch assemblies in such a manner that cutoffs, scraps or debris of a punching operation can be avoided in order to overcome the above-discussed shortcomings of the known prior art.

In order to achieve this object and overcome the disadvantages of assemblies known in the art, according to the present invention, a catheter-punch assembly is proposed, comprising a hollow outer tube portion extending in a longitudinal direction between a first end and a second end, a cable element extending inside the tube portion in a slidable manner, an actuation element provided at the first end of the tube portion and adapted to cause a sliding motion of the cable element between a first position and a second position upon actuation by a user, and a punch element, which is connected to or formed integrally with the cable element, the punch element in turn comprising a base portion adjacent to the cable element, a bridge portion extending from the base portion at a section of its circumference in the longitudinal direction, and a head portion comprising a first longitudinal section extending from the bridge portion in the longitudinal direction, a second longitudinal section opposite to the first longitudinal section and extending along a part of the longitudinal extension of the first longitudinal section, and a longitudinal end section connecting the first and second longitudinal sections, wherein a single punch section is provided between the first and second longitudinal sections, in particular at one circumferential side thereof, with a clearance further provided between the first and second longitudinal sections, wherein in the first position of the cable element, the punch section of the punch element is located outside the second end of the tube portion, and in the second position of the cable element, the punch section is located inside the tube portion.

Thus, by providing a single punch section between two longitudinal sections of a head portion of a punch element in an assembly according to the present invention together with a clearance between the remainder of the longitudinal sections, an element to be punched or cut with said assembly will be separated at only a single position and no cutoff, scrap or debris will result from such a punching operation. Thus, the risk of jamming the device will be avoided in that the two parts of the punched element will easily separate from one another after the punching operation as they are moved away from one another as well as from the punch element of the assembly. Furthermore, providing the clearance and thus reducing the size of the single punch sections increases the force per area acting on the element to be punched. In particular, compared with punch elements comprising two comparable punch sections, the punching force may be doubled without producing scrap or debris.

Due to the design and layout of the punch element, opposite to the single punch section in a radial direction of the punch element, a clearance is formed and no component is located, such that the object to be punched can easily be caught and separation of the two resulting parts of the punched element after the punching operation is ensured. The punching operation itself will take place between the front end face of the tube portion at its second end and the single punch section translating from its first position to its second position upon actuation of the actuation element by a user and corresponding transmission of said actuation by means of the cable element.

In order to facilitate the punching operation, the punch section may be slanted towards the end section going from the second longitudinal section towards the first longitudinal section such that after being caught and prior to being punched, the corresponding tubular or wire-shaped element will be guided between the first longitudinal section and the single punch section of the punch element. In this context, the second longitudinal section may also be slanted in an extension of the punch section to further facilitate said catching of the element to be punched before performing the punching operation.

While different geometries of both the tube portion and the punch element are conceivable in the context of the present invention, the base portion of the punch element may in particular have an outer shape substantially corresponding to the inner shape of the tube portion such that it will be guided along the inner walls thereof for reducing the risk of jamming the assembly and to increase precision of its handling.

In a similar manner, the first longitudinal section, the second longitudinal section and/or the end section of the punch element may have an outer shape substantially corresponding to the inner shape of the tube portion.

As already briefly mentioned above, the tube portion itself may in principle have an arbitrarily shaped cross section and it is for example conceivable to form its cross section in a square manner in order to avoid rotation of the punch element inside the tube portion. On the other hand, the tube portion may also have a substantially circular inner and/or outer cross section which may allow it for the punch element to be rotatable inside the tube portion in particular in a manual manner by the corresponding operator.

To further improve the punching ability of the assembly according to the present invention, the tube portion at its second end may have a tapered cross section such that by the interaction of said second end face of the tube portion and the punch section of the punch element, a separation of the element to be punched is simplified.

While the tube portion may be made of a rigid material and as such not be bendable, in certain embodiments the tube portion may also be made of a flexible material in order to allow for a bending of the catheter assembly during its use.

Also, the actuation element of the assembly may comprise an elastic element which is adapted to bias the cable element towards its second position in order to facilitate the handling of the assembly for a user. On the other hand, different embodiments of actuation elements may be provided in assemblies according to the present invention, including electronically controlled electromechanical devices with motors and purely manual components in which the translation of the cable element between its first and second positions is affected by manually moving a handle element.

According to a further aspect, the present invention relates to a system, comprising an assembly according to the invention as just described, and a tubular or wire-shaped element to be punched by the punch element, wherein in the first position of the cable element, the distance between the longitudinal end of the second longitudinal section of the punch element and the second end of the tube portion is slightly larger than the diameter of the tubular or wire-shaped element to be punched. This choice of dimensions of both the punch element and the element to be cut facilitates operation of the assembly for a user and reduces the risk of catching tissue unrelated to the element to be punched inside the punch element.

Typically, said tubular or wire-shaped elements may have a diameter of 0.2 mm to 3.0 mm and/or may be made of a plastic or metal material.

In one particular use case, inflatable tubular elements and/or elements filled with a substance may be used in the system according to the invention which may serve as inflatable implants.

Further features and advantages of the present invention will become even clearer from the following description of an embodiment thereof, when viewed together with the accompanying drawings. These show in particular:
- Figures 1a and 1b: two transparent views of a punch element of a catheter-punch assembly according to the present invention;
- Figure 1c: an isolated view of the punch element shown in figures 1a and 1b; and
- Figure 2: the actuation element of the assembly of figures 1a and 1b.

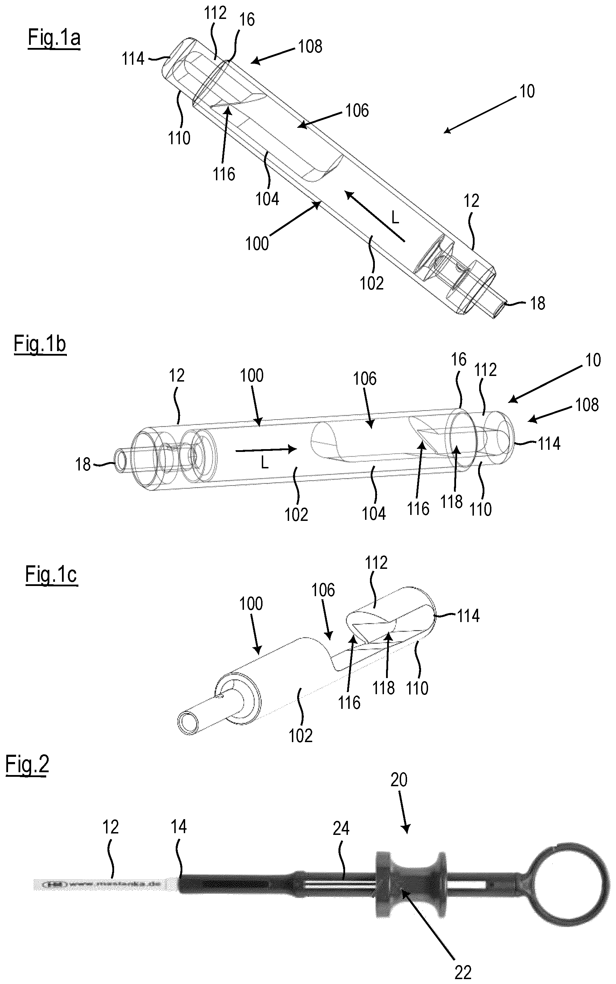

Figures 1a and 1b in two different views show a punch element 100 of a catheter-punch assembly 10, of which further components and in particular an actuation element are shown in figure 2. Furthermore, an isolated view of the punch element 100 is shown in figure 1c.

Said catheter-punch assembly 10 in addition to the punch element 100 described below comprises a hollow outer tube portion 12 extending in a longitudinal direction between a first end 14 and a second end 16, wherein the tube portion 12 is made from an elastic material and is bendable, such that the longitudinal direction L thereof is not understood to be perfectly straight in all configurations but rather merely extends between the two ends 14 and 16.

Inside said hollow outer tube portion 12, a cable element 18 is housed in a slidable manner and extends between the punch element 100 shown in figures 1a and 1b on the second end 16 of the tube portion 12 and an actuation element 20 shown in figure 2 and provided at the first end 14 of the tube portion 12. Herein, the actuation element 20 is adapted to cause a sliding motion of the cable element 18 between first and second positions upon manual actuation by a user, wherein said manual actuation is performed by displacing a grip element 22 against the action of a spring element 24, such as a helical spring, which biases the cable element 18 towards its second position further discussed below.

On the other hand, the punch element 100 shown in figures 1a to 1c is connected to the cable element 18 and as such may slide inside the tube portion 12 and partially out of it upon a translation of the cable element 18 effected by the actuation of the actuation element 20 by a user as just described.

The punch element 100 comprises a base portion 102 with a circular cross section which substantially corresponds to the inner shape of the tube portion 12. Extending from said base portion 102, the punch element 100 further comprises a bridge portion 104 at a section of its outer circumference in the longitudinal direction L, wherein opposite to the bridge portion 104, a clearance 106 is formed, by means of which an element or object to be punched with the punch element may be caught as will be discussed below.

Furthermore, the punch element 100 comprises a head portion 108, in turn comprising a first longitudinal section 110 extending from the bridge portion 106 in the longitudinal direction L, a second longitudinal section 112 opposite to the first longitudinal section 110 and extending along a part of the longitudinal extension of the first longitudinal section 110 terminating at the clearance 106 mentioned above.

Furthermore, the head portion 108 also comprises a longitudinal end section 114 connecting the first and second longitudinal sections 110 and 112, wherein a single punch section 116 is provided at one circumferential side between the first and second longitudinal sections 110, 112. Opposite to the single punch section 116, a further clearance 118 is formed such that during the punching operation discussed below, no cutoffs or scraps of the element to be punched will result, while increasing the force per area acting on the element to be punched.

A punching operation of the punch element shown in figures 1a to 1c is performed such that by means of an operation of the operating element 20, the cable element is translated to its first position, in which the punch section 116 of the punch element 100 is located outside the second end 16 of the tube portion 12 such that an element to be punched can be caught in the clearance 106 opposite to the bridge portion 104. By means of the slanted shape of the punch section 116 towards the end section 114 going from the second longitudinal section 112 towards the first longitudinal section 110, said element to be punched will be remain caught once the cable element 18 is translated to its second position shown in figures 1a and 1b in which the punch section 116 is located inside the tube portion 12 as shown in figures 1a and 1b. Thus, the element to be punched will be separated due to the interaction between the single punch section 116 and the face of the tube portion 12 at its second end 16.

## Claims

1. Catheter-punch assembly (10), comprising:
- a hollow outer tube portion (12) extending in a longitudinal direction (L) between a first end (14) and a second end (16);
- a cable element (18) extending inside the tube portion (12) in a slidable manner;
- an actuation element (20) provided at the first end (14) of the tube portion (12) and adapted to cause a sliding motion of the cable element (18) between a first position and a second position upon actuation by a user; and
- a punch element (100), which is connected to or formed integrally with the cable element (18), comprising:
∘ a base portion (102) adjacent to the cable element (18);
∘ a bridge portion (104) extending from the base portion (102) at a section of its circumference in the longitudinal direction (L); and
∘ a head portion (108) comprising a first longitudinal section (110) extending from the bridge portion (108) in the longitudinal direction (L), a second longitudinal section (112) opposite to the first longitudinal section (110) and extending along a part of the longitudinal extension of the first longitudinal section (110), and a longitudinal end section (114) connecting the first and second longitudinal sections (110, 112),
wherein a single punch section (116) is provided between the first and second longitudinal sections (110, 112) with a clearance (118) further provided between the first and second longitudinal sections (110, 112),
wherein in the first position of the cable element (18), the punch section (116) of the punch element (100) is located outside the second end (16) of the tube portion (12), and in the second position of the cable element (18), the punch section (116) is located inside the tube portion (12).

2. Assembly (10) according to claim 1,
wherein the single punch section (116) is positioned at one circumferential side between the first and second longitudinal sections (110, 112).

3. Assembly (10) according to claim 1 or 2,
wherein the punch section (116) is slanted towards the end section (114) going from the second longitudinal section (112) towards the first longitudinal section (110).

4. Assembly (10) according to claim 3,
wherein the second longitudinal section (112) is also slanted in an extension of the punch section (116).

5. Assembly (10) according to any of the preceding claims,
wherein the base portion (102) of the punch element (100) has an outer shape substantially corresponding to the inner shape of the tube portion (12).

6. Assembly (10) according to any of the preceding claims,
wherein the first longitudinal section (110), the second longitudinal section (112) and/or the end section (114) of the punch element (100) have an outer shape substantially corresponding to the inner shape of the tube portion (12).

7. Assembly (10) according to any of the preceding claims,
wherein the tube portion (12) has a substantially circular inner and/or outer cross section.

8. Assembly (10) according to any of the preceding claims,
wherein the punch element (100) is rotatable inside the tube portion (12).

9. Assembly (10) according to any of the preceding claims,
wherein the tube portion (12) at its second end (16) has a tapered cross section.

10. Assembly (10) according to any of the preceding claims,
wherein the tube portion (12) is made of a flexible material.

11. Assembly (10) according to any of the preceding claims,
wherein the actuation element (20) comprises an elastic element (24) which is adapted to bias the cable element (18) towards its second position.

12. System, comprising an assembly (10) according to any of the preceding claims and a tubular or wire-shaped element to be punched by the punch element (100), wherein in the first position of the cable element (18), the distance between the longitudinal end of the second longitudinal section (112) of the punch element (100) and the second end (16) of the tube portion (12) is slightly larger than the diameter of the tubular or wire-shaped element.

13. System according to the preceding claim,
wherein the tubular or wire-shaped element has a diameter of 0.2 to 3.0 mm.

14. System according to any of claims 12 and 13,
wherein the tubular or wire-shaped element is made of a plastic or metal material.

15. System according to any of claims 12 to 14,
wherein the tubular element is inflatable and/or filled with a substance.
